**Europäisches Patentamt**

(19) **European Patent Office**

**Office européen des brevets**

(11) Publication number: **0 000 986**
**B1**

(12) **EUROPEAN PATENT SPECIFICATION**

(45) Date of publication of patent specification: **07.04.82**

(21) Application number: **78300240.5**

(22) Date of filing: **03.08.78**

(51) Int. Cl.³: **A 61 N 1/36, G 08 C 19/22**

(54) Programmer for implanted pacer.

(30) Priority: **19.08.77 GB 3491277**
**19.06.78 US 917139**

(43) Date of publication of application:
**07.03.79 Bulletin 79/5**

(45) Publication of the grant of the patent:
**07.04.82 Bulletin 82/14**

(84) Designated Contracting States:
**DE FR GB NL SE**

(56) References cited:
**LU - A - 76 880**
**US - A - 3 906 348**
**US - A - 4 024 875**
**US - A - 4 049 004**

(73) Proprietor: **BIOTRONIK Mess- und Therapiegeräte**
**GmbH & Co Ingenieurbüro Berlin**
**Sieversufer 8**
**D-1000 Berlin 47 (DE)**

(72) Inventor: **Keller, John Walter**
**8600 S.W. 54th Avenue**
**Miami Florida (US)**
Inventor: **Digby, Dennis**
**2409 Gunflint Trail**
**Brooklyn Park Minnesoita 55444 (US)**
Inventor: **Coombes, Alan**
**8103 28th Avenue North**
**New Hope Minnesota 55427 (US)**

(74) Representative: **Christiansen, Henning, Dipl.-Ing.**
**Unter den Eichen 108a**
**D-1000 Berlin 45 (DE)**

Courier Press, Leamington Spa, England.

Programmer for implanted pacer

Field of the invention

This invention relates to an apparatus for transmitting a preselected signal comprising a sequence of signal bits of different values. Such an apparatus can be termed a programmable transmitter where the signal it is desired to transmit to a receiver may be selected, or set, prior to transmission. The invention is especially concerned with programmable transmitters for enabling programming signals to be transmitted to, and received by, implanted, programmable body function control apparatus such as cardiac pacemakers.

Background art

Pacemakers for generating artificial stimulating pulses for the heart, and which may be implanted in the body, are well known. Originally the electrical circuitry for such pacemakers was of analog design, but in recent years digital circuitry has been also employed. A digital approach to pacemakers has led to the evolution of programmable pacemakers— pacemakers having parameters such as pulse rates which are adjustable (programmable) once the pacemaker has been implanted. Programmable pacemakers are described in, for instance, British Specifications 1,385,954 and 1,398,875. Such pacemakers have circuitry to detect and decode signals transmitted outside the body and alter the program accordingly. In British Specification 1,385,954 (claiming priority based on U.S.S.N. 141694, in turn a parent of U.S.P.N. 3,805,796 to Tenz) the programming is accomplished by means of a magnetic field which is sensed by a magnetic reed switch; the opening and closing of the switch provides programming pulses to a program store. In British Specification 1,398,875 (based on U.S.P.N. 3,833,005 to Wingrove) the programming is by means of radio frequency transmission and reception.

Although much attention has been paid to the development of the implantable programmable pacemaker, there is also a need for an external programmable transmitter which can be programmed with the pacemaker program and this program then accurately transmitted to the implanted pacemaker. Prior art proposals for external, non-invasive, pacemaker programmers have been set forth in, for example, US Patents 4,024,875 and 4,049,004.

We have now developed a programmable transmitter for such use.

According to the invention we provide an external programmer for supplying a fixed number of signal bits as a program for varying given operational functions of an implanted, programmable body function apparatus comprising:

register means (5, 25) for preselecting and storing a fixed number of signal bits, said bits being a coded representation of the program to be supplied serially to said implanted apparatus,

means (4) for generating a pulse signal at a predetermined frequency, and

output means (13, 23) for transmitting signals from said generating means (4) to said implanted apparatus in response to the preselected and stored signal bits, *characterised in that* the programmer additionally comprises:

counter means (3) responsive to said generating means (4) for providing digital control states at three sub-intervals of a datum count, and

means (11; 19, 20) responsive to said counter means (3), for periodically enabling said output means (13, 23) to transmit signals at the rate of the smallest of the sub-intervals, the periodicity of said enabling means corresponding to a fixed fractional part of said datum count period, and for disabling said output means (13, 23), once for each periodic enabling at a time after each enabling, each said time successively corresponding to the logical state of each associated successive one of said signal bits.

Brief description of the drawing

Preferred features of the invention will now be described, by way of example, with reference to the accompanying drawings, in which:

Figure 1 shows schematically the electrical circuit diagram of an external programmer for encoding an 8-bit program, and for transmitting the program to an implanted, programmable cardiac pacemaker.

Figure 2 is a timing diagram for use with Figure 1, and

Figure 3 shows schematically the electrical circuit diagram of an alternative embodiment.

Best mode of carrying out the invention

The illustrated programmer is designed to transmit an 8-bit tone burst modulated program, that is, an 8-bit pulse width modulated signal where a sine wave carrier frequency is pulse width modulated by each of the 8 data bits.

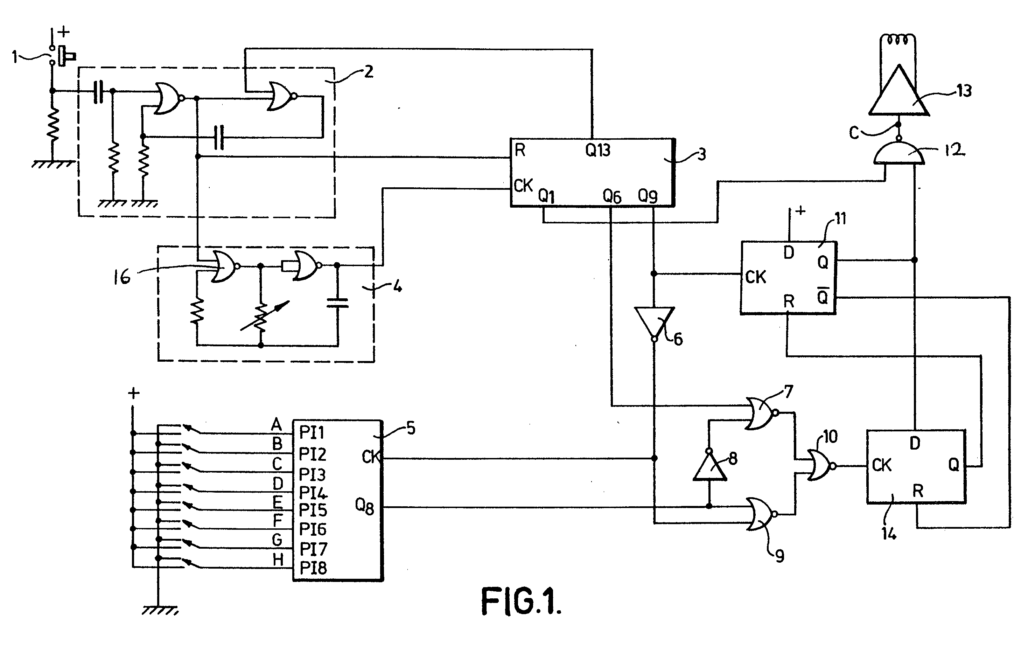

Referring to Figure 1, the programmer comprises a "press-to-program" switch 1 tied between the positive supply rail and earth. The switch output drives a monostable 2. The monostable 2 is employed to reset a 13 stage counter 3, and to control an oscillator 4. The oscillator 4 includes an "oscillator disable" NOR gate 16 on its input side which, when supplied with a high input from monostable 2, provides a low output which disables the oscillator output to a clock input of counter 3.

Only the Q1, Q6, Q9, and Q13 stages of counter 3 are employed. stage Q9 is employed to clock a parallel in/serial out eight bit shift register 5 via an inverter 6. The eight parallel inputs for shift register 5 (PI 1 to 8) are labelled A to H and the values of such inputs are set by eight switches which individually can be connected to the positive supply rail or to earth.

The outputs from the Q6 stage of counter 3, inverter 6, and the serial output Q8 of shift register 5 drive a pulse steering network composed of NOR gates 7, 9 and 10 and inverter 8.

The Q9 stage of counter 3 also clocks a D-type flip-flop 11 whose D-input is tied to the positive supply rail. The Q output of flip-flop 11 is supplied to one input of a NAND gate 12, whose second input is supplied by the Q1 stage of counter 3. NAND gate 12 drives an amplifier/transmitter 13, outputs from which are transmitted into the body for receipt by an implanted cardiac pacemaker where the received pulses are decoded and employed for changing the program stored in the pacemaker.

The output of NOR gate 10 clocks a further D-type flip-flop 14, whose D-input and reset are driven by the Q and $\overline{Q}$ outputs, respectively, of flip-flop 11. The Q output of flip-flop 14 is employed to reset flip-flop 11.

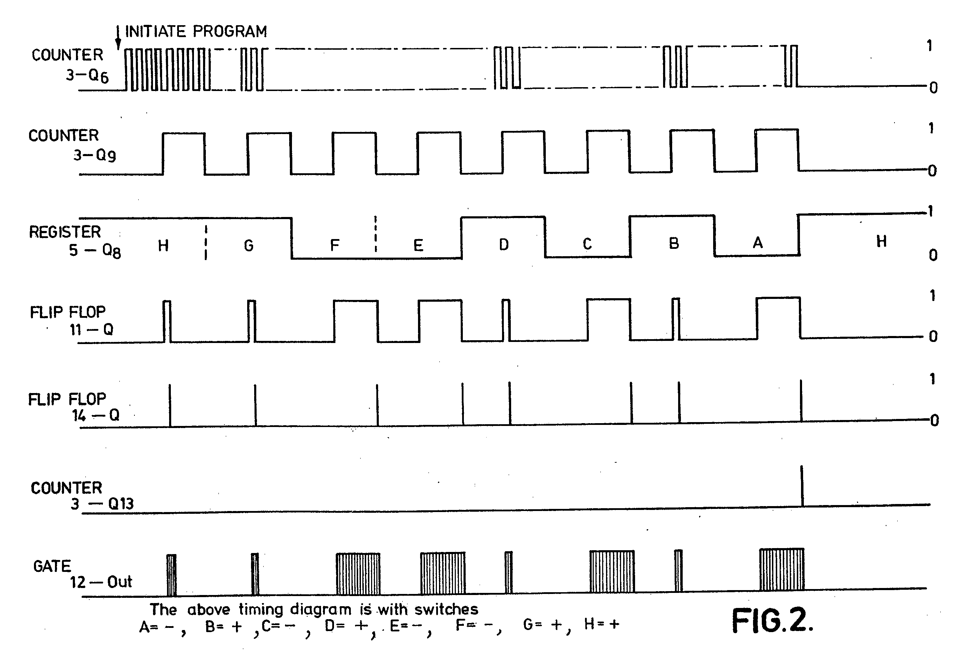

The operation of the programmer will now be described generally and then in detail with reference also to the timing diagram of Figure 2, which illustrates the pulses at positions indicated on Figure 1.

The pacemaker program transmitted by amplifier/transmitter 13 consists of an eight bit pulse-width modulated signal wherein each pulse is itself several cycles of a sine wave carrier frequency. The pulses of short bit length (which might represent "0" in the pacemaker program) and long bit length ("1") are determined by the signal supplied to NAND gate 12 from flip-flop 11 and are initially generated by the values selected by the settings of the eight switches on the inputs A to H of shift register 5. The carrier frequency present in each pulse is derived from the rapid transitions made by the Q1 stage of counter 3, as supplied to the second input of NAND gate 12. The output of NAND gate 12 is thus the square wave carrier frequency from Q1 modulated by the long or short bit lengths determined by the switching times of flip-flop 11. After passage through amplifier/transmitter 13, this output becomes the tone burst modulated signal described above.

Referring to the operation in more detail, with the press-to-program switch normally open, the monostable 2 output remains high maintaining a reset on counter 3 and disabling oscillator 4.

When it is desired to change the 8-bit program stored in the implanted pacemaker, the values of the 8-bits are selected by providing an appropriate combination of values through opening or closing each of the eight switches on the inputs to shift register 5. In the example given, referring to Figure 2, the inputs H, G, D, and B are tied to the positive supply rail whereas inputs F, E, C, and A are earthed. This will provide, as to be described, for transmission by amplifier/transmitter 13, the program 00110101 where "0" represents a pulse of short length (and "1" of long length), it being appreciated that the values are transmitted in the sequence HGFEDCBA. This arises since the first value appearing at the Q8 output of shift register 5 is the value of the PI 8 input (H).

To transmit the selected program, the press-to-program switch 1 is depressed. This causes monostable 2 to fire (i.e. causing its output to go low), thus removing the reset from counter 3 and allowing oscillator 4 to commence running at a period $T_0$ determined by its timing components. Counter 3 will increment by 1 count on every negative clock edge provided by oscillator 4.

At a count of Q9, flip-flop 11 clocks the level of its D-input to its Q output, putting Q "high" and $\overline{Q}$ "low". This removes the reset on flip-flop 14 and places its D-input "high". The "high" output of Q is also applied to NAND gate 12 to commence transmission of the first bit in the 8-bit sequence of data. The next positive edge on the clock input of flip-flop 14 will clock its Q output to "high" thus resetting flip-flop 11. The reset of the latter will, in turn, reset flip-flop 14, causing its Q output to go "low" and terminate transmission of the first bit in the 8-bit sequence.

The time at which flip-flop 14 clocks a "high" to its Q output determines the width of the flip-flop 11 Q output and hence the pulse width of the modulation at point C in Figure 1. It is dependent upon the state of the steering network comprising components 7, 8, 9, and 10. The level on the Q8 output of shift register 5 determines whether the clock pulse for flip-flop 14 arises via NOR gate 7 or 9 and is derived from either the Q6 or Q9 stage of counter 3. With gate 7 enabled, the first positive edge on the flip-flop 14 clock input occurs at $32T_0$ from the time the flip-flop 11 Q output went "high". With gate 9 enabled, this first positive edge occurs at $256T_0$ from the time the flip-flop 11 Q output went "high".

Initially, the Q8 output of shift register 5 represents the value at its H input ("high") and this causes the pulse supplied by flip-flop 11 to NAND gate 12 to be cut off after $32T_0$ (i.e. causing a short pulse representing "0" to be transmitted).

Subsequent bits are then transmitted as the values of the seven remaining shift register stages are sequentially clocked to the Q8 output of shift register 5. The bits are transmitted at each positive transition of the Q9 stage of counter 3 until Q13 is reached. Shift register 5 is clocked on each negative edge of the Q9 stage of counter 3. When the Q13 stage of counter 3 goes high this resets monostable 2 to its initial condition, resets counter 3 and disables the oscillator 4. By this time the values of all eight stages of shift register 5 will have appeared at the Q8 output and the corresponding 8-bits will have been transmitted by amplifier/transmitter 13.

In a typical programmer constructed as described, $T_0$ was set to 0.05 ms: this provided short pulses of 1.6 ms in width and long pulses of 12.8 ms in width. These pulses were modulated via the Q1 stage of counter 3 at 10 KHz.

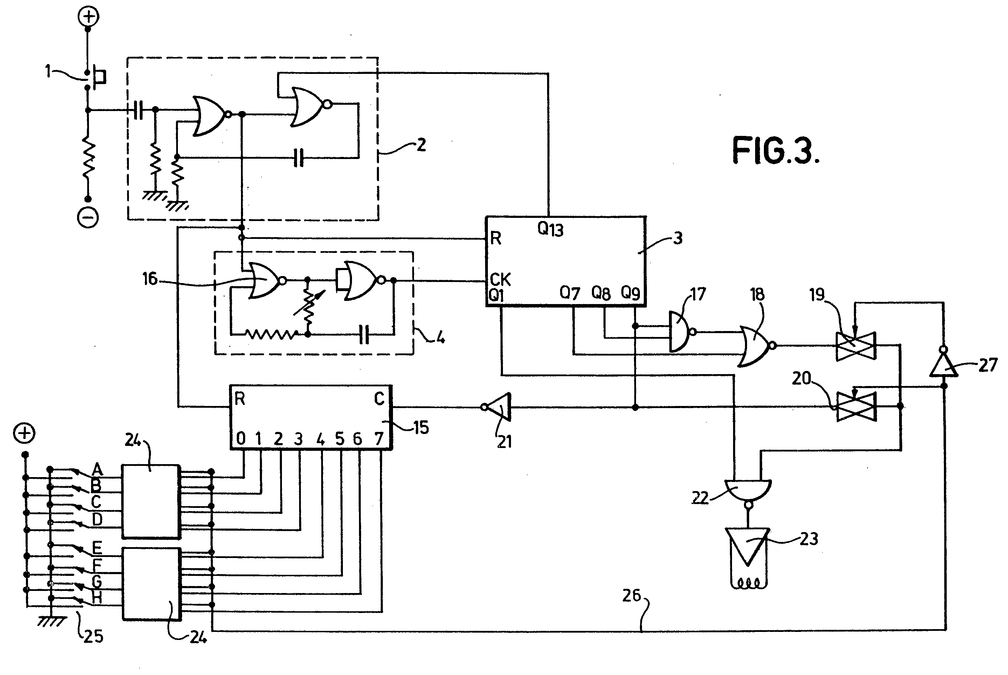

The alternative embodiment shown in Figure 3 will now be described.

Referring to Figure 3, the programmer again comprises a "press-to-program" switch 1 tied between the electrical supply rails. The switch output drives a monostable 2. The monostable 2 is employed to reset a 13 stage counter 3 to control an oscillator 4, and to reset a decade counter 15. The oscillator 4 includes an "oscillator disable" NOR gate 16 which, when supplied with a high input from monostable 2, provides a low output which disables the oscillator output to a clock input of counter 3.

The Q7, 8 and 9 output stages of counter 3 are combined through a NAND gate 17 and a NOR gate 18 to provide a "short" output pulse to a transmission gate 19. The Q9 output stage of counter 3 provides a "long" output pulse to a transmission gate 20 and, through inverter 21, to the clock input of the decade counter 15.

A NAND gate 22 receives the Q1 output of counter 3 and also the outputs of transmission gates 19 and 20. The output of NAND gate 22 drives an amplifier/transmitter 23, output pulses from which are transmitted into the body for receipt by an implanted programmable cardiac pacemaker where the received pulses are decoded and employed for changing the program stored in the pacemaker.

The Q13 output of counter 3 is supplied to a disable input of monostable 2.

The decade counter 15 has eight output stages (0 to 7) which are successively employed to control (turn on and off) eight transmission gates which, in this embodiment, take the form of two quad/bilateral switches 24. The eight gates formed by the switches 24 each receive an input from a selected switch 25. The switches 25 ("A" to "H") can be individually connected to the positive supply rail or to earth. The eight outputs of the switches 24 are coupled together to provide a common bus 26 which is employed to control transmission gate 20 and (via inverter 27) transmission gate 19.

The operation of the programmer will now be described first generally and then in more detail.

The counts which are supplied by counter 3 to transmission gates 19 and 20 supply pulses representative of short bit lengths (e.g. "0") and long bit lengths (e.g. "1"). The eight bit program desired is selected by setting the switches 25 appropriately and these values are sequentially clocked onto the common bus 26 to open either transmission gate 19 or 20 depending upon the logic value supplied on bus 26. The short or long pulse supplied to the amplifier/transmitter 23 from the opened transmission gate 19 or 20 is employed to modulate the higher frequency count supplied to NAND gate 22 from the Q1 stage of counter 3 so that the output of the amplifier/transmitter is an eight-bit tone burst signal.

Referring to the operation in more detail, with the press to program switch 1 normally open, the monostable 2 output remains high, maintaining a reset on counters 3 and 15 and disabling oscillator 4. No output is provided to amplifier/transmitter 23. When it is desired to change the 8-bit program stored in the implanted pacemaker, the values of the 8-bits are selected by providing an appropriate combination of values through opening or closing each of the switches 25. The press to program switch 1 is then pressed. Pressing, i.e. closing, switch 1 causes monostable 2 to fire (i.e. causing its output to go low), thus removing the reset from counters 3 and 15 and allowing oscillator 4 to commence running. The removal of the reset to decade counter 15 leaves a count at its "zero" stage, which opens the transmission gate in switches 24 corresponding to switch 25 "A". The output of switch 25 "A" is supplied on common bus 26 and depending on its logic value, opens either transmission gate 19 or 20 thus to supply either a short pulse or a long pulse to NAND gate 22. This short or long pulse modulates the high frequency output of the Q1 stage of counter 3, and is amplified and transmitted by amplifier/transmitter 13.

As soon as the Q9 stage of counter 3 falls, decade counter 15 is incremented by one so that the count, now at its "one" stage, causes the output value supplied by switch 25 "B" rather than 25 "A" to control which of the transmission gates 19 and 20 is opened. The short or long pulse (modulating the Q1 high frequency output as before) is again transmitted by amplifier/transmitter 23.

This cycle repeats for each of the eight switches 25 so that a total of eight tone burst modulated pulses, of either short or long width, is transmitted. Once the eighth pulse has been transmitted, the Q13 stage of counter 3 is then reacted to disable monostable 2. This causes the latter to revert to its stable state, which reintroduces a reset to counters 3 and 15 and disables oscillator 4. No further information is hence transmitted until the press to program switch 1 is once again depressed, when the whole cycle would then repeat.

Typically, the oscillator has a 20 KHz frequency. This provides a 10 KHz output at the Q1 stage of counter 3, a pulse length of 3.2 ms to transmission gate 19 (a "short" pulse), a pulse length of 12.8 ms to transmission gate 20 (a "long" pulse), and a data rate for clocking decade counter 15 of 25.6 ms. The NAND gate 22 thus transmits to amplifier/transmitter 23 eight bits, either of 3.2 ms or 12.8 ms in width and each including a 10 KHz square wave carrier.

## Claims

1. An external programmer for supplying a fixed number of signal bits as a program for varying given operational functions of an implanted programmable body function apparatus comprising:

register means (5, 25) for preselecting and storing a fixed number of signal bits, said bits being a coded representation of the program to be supplied serially to said implanted apparatus,

means (4) for generating a pulse signal at a predetermined frequency, and

output means (13, 23) for transmitting signals from said generating means (4) to said implanted apparatus in response to the preselected and stored signal bits, characterised in that the programmer additionally comprises:

counter means (3) responsive to said generating means (4) for providing digital control states at three sub-intervals of a datum count, and

means (11; 19, 20), responsive to said counter means (3), for periodically enabling said output means (13, 23) to transmit signals at the rate of the smallest of the sub-intervals, the periodicity of said enabling means corresponding to a fixed fractional part of said datum count period, and for disabling said output means (13, 23), once for each periodic enabling at a time after each enabling, each said time successively corresponding to the logical state of each associated successive one of said signal bits.

2. A programmer according to Claim 1, characterised in that said disabling means comprises means (7—10; 19, 20), responsive to each occurrence of a given signal bit in said register means (5, 25), for disabling said output means (13, 23) upon the occurrence of the intermediate one of said sub-intervals and responsive to each occurrence of a signal bit of opposite logic in said register means (5, 25) for disabling said output means (13, 23) upon the occurrence of the third of said sub-intervals.

3. A programmer according to Claim 1 or 2, characterised in that said enabling and disabling means comprises two transmission gates (19, 20), said counter means (3) supplying inputs to said gates and the outputs supplied to said output means (23) by said gates being controlled by said preselected and stored signal bits.

4. A programmer according to Claim 1 or 2, characterised in that said enabling and disabling means comprises a first flip-flop (11) for controlling the time of commencement of transmission of any signal bit from the output means (23) and a second flip-flop (14) for controlling the time of termination of transmission of any signal bit from the output means (23).

5. A programmer according to any of Claims 1 to 4 characterised in that said generating means comprises:

programmer-activating switch means (1), monostable means (2), energised by said switch means for resetting said counter (3), and

oscillator means (4), enabled by said monostable means (2), for clocking said counter (3).

6. A programmer according to any of Claims 1 to 5, characterised in that said counter means (3), upon achievement of said datum count (Q13) suppresses said generating means (4).

## Patentansprüche

1. Externe Programmiervorrichtung zur Abgabe einer vorgebenen Anzahl von Signal-Bits als Programm zur Veränderung der Betriebsfunktionen einer implantierten programmierbaren Körperfunktionsvorrichtung mit

Registriermitteln (5, 25) zum Auswählen und Speichern einer vorgegebenen Anzahl von Signal-Bits, wobei diese Bits eine codierte Darstellung des Programms bilden, welches der implantierten Vorrichtung seriell zugeführt werden soll,

Signalerzeugungsmitteln (4) zum Erzeugen von Impulsen mit einer vorgegebenen Frequenz und

Ausgangsmitteln (13, 23) zur Übertragung von Signalen von den Signalerzeugungsmitteln (4) zur implantierten Vorrichtung in Abhängigkeit von den ausgewählten und gespeicherten Signal-Bits, dadurch gekennzeichnet, daß die Programmiervorrichtung weiterhin enthält:

Zählmittel (3), welche von den Signalerzeugungsmitteln (4) angesteuert werden, um digitale Kontrollzustände bei drei Unterintervallen eines Bezugzählerstandes zu erzeugen, und

Mittel (11, 19, 10), welche auf die Zählmittel (3) ansprechen, um die Ausgangsmittel (13, 23) zum Aussenden von Signalen, mit der Rate des kleinsten der Unterintervalle zu aktivieren, wobei die Periodizität der Aktiviermittel einem festen Bruchteil der Bezugszählerstände entspricht, und zum Deaktivieren der Ausgangsmittel (13, 23) jeweils einmal nach dem periodischen Aktivieren zu einer Zeit nach der Aktivierung, wobei jede der aufeinanderfolgenden Zeiten dem logischen Zustand eines zugeordneten, folgenden Signal-Bits entspricht.

2. Programmiervorrichtung nach Anspruch 1, dadurch gekennzeichnet, daß die Deaktivierungsmittel weitere Mittel (7 bis 10, 19, 20) enthalten, welche auf das Auftreten eines vorgebenen Signal-Bits in den Registermitteln (5, 25) hin ansprechen, um die Ausgangsmittel (13, 23) auf das Auftreten eines mittleren der Unterintervalle zu deaktivieren und auf das Auftreten von Signal-Bits entgegengesetzter Logik in den Registermitteln (5, 25) reagieren, um die Ausgangsmittel (13, 23) beim Auftreten des dritten der genannten Unterintervalle zu deaktivieren.

3. Programmiervorrichtung nach einem der Ansprüche 1 oder 2, dadurch gekennzeichnet, daß die Aktivierungs- und Deaktivierungsmittel zwei Übertragungsgatter (19, 20) enthalten, wobei der Zähler (3) Eingangssignalle für diese Gatter liefert und die Ausgangssignale, welche von diesen Gattern zu den Ausgangsmitteln (23) gelangen, durch die ausgewählten und gespeicherten Signal-Bits gesteuert werden.

4. Programmiervorrichtung nach einem der Ansprüche 1 oder 2, dadurch gekennzeichnet, daß die Aktivier- und Deaktiviermittel ein erstes Flip-Flop (11) enthalten, zum Steuern der Zeit der Fortsetzung der Übertragung eines Signal-Bits durch die Ausgangsmittel (23) und ein zweites Flip-Flop (14) zur Steuerung der Zeit der Beendigung der Übertragung irgendeines Signal-Bits durch die Ausgangsmittel (23).

5. Programmiervorrichtung nach einem der Ansprüche 1 bis 4, dadurch gekennzeichnet, daß die Signalerzeugungsmittel aufweisen:

Schaltmittel (1) zur Programmier-Aktivierung,

monostabile Mittel (2) welche durch die Schaltmittel angesteuert werden, zum Zurücksetzen des Zählers (3) sowie

Oszcillatormittel (4) welche durch die monostabilen Mittel (2) zum Takten des Zählers (3) angesteuert werden.

6. Programmiervorrichtung nach einem der Ansprüche 1 bis 5, dadurch gekennzeichnet, daß die Zählmittel (3) nach Erreichen des vorgegebenen Zählerstandes (Q 13) die Signalerzeugungsmittel (4) sperren.

## Revendications

1. Programmateur externe destiné à fournir un nombre fixe de bits de signal en tant que programme pour varier des fonctions opérationnelles données d'un appareil de fonction corporelle programmable implanté, programmateur qui comprend:

un dispositif à registre (5, 25) pour présélectionner et mémoriser un nombre fixe de bits de signal, ces bits étant une représentation codée du programme à fournir en série à l'appareil implanté,

un dispositif générateur (4) pour produire un signal pulsé à une fréquence prédéterminée, et

un dispositif de sortie (13, 23) pour transmettre des signaux du dispositif générateur (4) à l'appareil implanté en réponse aux bits présélectionnés et mémorisés, caractérisé en ce que la programmateur comprend en outre:

un dispositif compteur (3) sensible au dispositif générateur (4) pour fournir des états de commande numérique à trois sous-intervalles d'un comptage de transmission de donnée, et

un dispositif d'autorisation (11; 19, 20) sensible au dispositif compteur (3) pour périodiquement permettre au dispositif de sortie (13, 23) de transmettre des signaux au rythme du plus petit des sous-intervalles, la périodicité du dispositif d'autorisation correspondant à une fraction fixe de la période de comptage de donnée, et pour interdire le fonctionnement du dispositif de sortie (13, 23) une fois pour chaque autorisation périodique, à un instant intervenant après chaque autorisation, chacun de ces instants correspondant successivement à l'état logique du bit associé à la succession des bits du signal.

2. Programmateur selon la revendication 1, caractérisé en ce que le dispositif d'autorisation et d'interdiction comprend des dispositifs (7—10; 19, 20) sensibles à chaque apparition d'un bit de signal donné dans le dispositif à registre (5, 25) pour interdire le fonctionnement du dispositif de sortie (13, 23) lorsque se produit le sous-intervalle intermédiaire et sensibles à chaque apparition d'un bit de signal d'état logique contraire dans ce dispositif à registre (5, 25) pour interdire le fonctionnement du dispositif de sortie (13, 23) lorsque se produit le troisiéme des sous-intervalles.

3. Programmateur selon la revendication 1 ou 2, caractérisé en ce que le dispositif d'autorisation et d'interdiction comprend deux portes de transmission (19, 20), le dispositif computer (3) fournissant des signaux d'entrée à ces portes et les signaux de sortie appliqués par elles au dispositif de sortie (23) étant commandés par les bits de signal présélectionnés et mémorisés.

4. Programmateur selon la revendication 1 ou 2, caractérisé en ce que le dispositif d'autorisation et d'interdiction comprend une première bascule (11) pour commander l'instant du commencement de transmission de tout bit de signal par le dispositif de sortie, ainsi qu'une deuxième bascule (14) pour commander l'instant de finition de la transmission de tout bit de signal par le dispositif de sortie (23).

5. Programmateur selon l'une quelconque des revendications 1 à 4, caractérisé en ce que le dispositif générateur comprend:

un dispositif de commutation (1) pour la mise en fonction du programmateur,

un dispositif monostable (2) excité par ce dispositif de commutation et destiné à remettre le dispositif compteur (3) à zéro, et

un dispositif oscillateur mis en action par le dispositif monostable (2) et servant d-horloge pour le dispositif compteur (3).

6. Programmateur selon l'une quelconque des revendications 1 à 5, caractérisé en ce que le dispositif compteur (3) provoque la mise hors fonction du dispositif générateur (4) lorsque le comptage de transmission de donnée est terminé (Q 13).

FIG.1.

0 000 986

The above timing diagram is with switches
A= − , B= + , C= − , D= + , E= − , F= − , G= + , H= +

FIG.2.

FIG.3.